# EUROPEAN PATENT SPECIFICATION

(11) **EP 1 518 863 B1**
(45) Date of publication and mention of the grant of the patent: **15.12.2010**
(21) Application number: 04292296.3
(22) Date of filing: 24.09.2004
(51) Int. Cl.: C07K 16/26, C07K 14/59, A61K 39/395

(54) **Antibodies modulating the activity of equine chorionic gonadotropin.**
Antikörper modulierende die biologische Aktivität von Pferd-Choriongonadotropin
Anticorps pour le modulation de l'activité de la gonadotrophine chorionique equine

(30) Priority: 26.09.2003 EP 03292365
(43) Date of publication of application: 30.03.2005
(73) Proprietor: INSTITUT NATIONAL DE LA RECHERCHE AGRONOMIQUE (INRA), 75007 Paris Cedex 07 (FR); CENTRE NATIONAL DE LA RECHERCHE SCIENTIFIQUE (CNRS), 75794 Paris Cedex 16 (FR); Universite Francois Rabelais de Tours, 37041 Tours Cedex 1 (FR)
(72) Inventor: Maurel, Marie-Christine, 37000 Tours (FR); Roy, Francois, 69001 Lyon (FR); Herve, Virginie, 37510 Ballan-Mire (FR); Bertin, Jean, 37260 Monts (FR); Guillou, Florian, 37000 Tours (FR)
(74) Representative: Vialle-Presles, Marie José

(56) References cited:
- EP-A- 0 265 156
- EP-A- 0 839 831
- MAUREL MARIE-CHRISTINE ET AL: "Immunochemical study of equine chorionic gonadotropin (eCG/PMSG): Antigenic determinants on alpha- and beta-subunits" BIOCHIMICA ET BIOPHYSICA ACTA, vol. 1159, no. 1, 1992, pages 74-80, XP009030538 ISSN: 0006-3002

## Description

The invention relates to the use of antibodies directed against equine chorionic gonadotropin (eCG), to modulate the activity of said hormone.

Gonadotropins play an important role in the control of gametogenetic and endocrine activities of gonads. These complex glycoproteins are composed of two dissimilar α and β, whose non-covalent association is required for their biological activities. Gonadotropins belong to the glycoprotein hormone family and comprise hypophysal luteinizing hormone (LH) and follicle-stimulating hormone (FSH), and human (hCG) and equine chorionic gonadotropin (eCG). Gonadotropins are currently used in human and veterinary medicine to mimic endocrine mechanisms of sexual cycles. In humans, FSH and hCG are used to conduct in-vitro fertilization (IVF) protocols and anovulation treatment. In mammals, particularly ruminants, eCG is commonly used, in association with a progestogen, in induction and synchronization of oestrus and ovulation treatments, and in superovulation treatments.

Equine chorionic gonadotropin (eCG) is secreted by trophoblastic cells in the mare between the 36th and 120th days of gestation (ALLEN and MOOR, J. Reprod. Fertil. 29: 313-316, 1972). Interestingly, in equine species dimeric eCG binds only to the LH receptor (GUILLOU and COMBARNOUS, Biochim. Biophys. Acta 755: 229-236, 1983; COMBARNOUS et al., Ann. Endocr. Paris 45: 261-268, 1984) whereas it exhibits pronounced FSH activity in addition to its LH activity in species other than equine (PAPKOFF, Biochem. Biophys. Res. Commun. 58: 397-404, 1974; COMBARNOUS et al., FEBS Lett. 90: 65-68, 1978; LICHT et al., J. Endocrinol. 83: 311-322, 1979). Each subunit is composed of a peptidic part connected to a glycannic moiety (N- and O- chains). eCG is the most heavily glycosylated glycoprotein hormone, with a majority of bi-antenna glycans ending mainly by sialic acids which play an important role in the hormone half-life in vivo (MATZUK et al., Endocrinol. 126: 376-383, 1990). Glycannic chains are also involved in the stability of the heterodimeric hormone (HEIKOOP et al., Eur. J. Biochem. 253: 354-356, 1998) and are necessary for the efficiency of the signal transduction (MATZUK et al., J. Biol. Chem. 264: 2409-2414, 1989; SAIRAM and JIANG, Mol. Cell Endocrinol. 85: 227-235, 1992). The dual activity of eCG, its long half-life and its availability in large quantities make this unique gonadotropin a convenient exogenous hormone in treatments to induce and synchronize oestrus and ovulation.

However, it has been reported that repeated use of eCG treatments for induction of ovulation is generally followed by decreased fertility which can decrease from 60% to 40% (BARIL et al., Theriogenol. 40: 621-628, 1993; BARIL et al., Reprod. Domest. Anim. 27: 161-168, 1992) and this phenomenon wax explained by unwanted immunological responses (ROY et al., Biol. Reprod. 60: 805-813, 1999). The presence of anti-eCG antibodies (anti-eCG antibodies) in the plasma of eCG-treated goats and ewes has been demonstrated (BARIL et al., 1993, aforementioned; BARIL et al., 1992, aforementioned; ROY et al., 1999, aforementioned; ROY et al., Biol. Reprod. 61: 209-218, 1999). A survey with 2500 females (ewes and goats) demonstrated that a 500 IU eCG injection induces strictly similar humoral immune response (HIR) kinetics, with a maximum anti-eCG antibody concentration between 10 and 17 days after injection (ROY et al., 1999, aforementioned; ROY et al., 1999, aforementioned). About 65% of treated females developed antibodies to eCG but anti-eCG antibody levels were highly variable among individuals. Previous reports showed a significant association between the females displaying high or low anti-eCG response phenotypes and particular microsatellite alleles located inside the Major Histocompatibility Complex (MHC) class II (ROY et al., 1999, aforementioned; ROY et al., 1999, aforementioned). Anti-eCG antibodies, already present by the time of eCG injection and resulting from the previous immune response against eCG one year earlier, are defined as: "residual anti-eCG antibodies". Females with a high residual anti-eCG antibody concentration exhibited a significantly lower fertility after artificial insemination (AI), because of a delay both in the onset of estrus and in the preovulatory LH surge (ROY et al., 1999, aforementioned; ROY et al., 1999, aforementioned).

Surprisingly, some females displayed a significant fertility after AI despite of high residual anti-eCG antibody levels. Therefore, to address the mechanism of immune interference on eCG-induced reproductive function, the inventors have tested the impact of anti-eCG antibodies on both LH and FSH bioactivities of the eCG. They have found that anti-eCG antibodies recognizing primarily the glycannic moiety of eCG, were able to modulate independently the LH and FSH bioactivities, i.e. to stimulate both LH and FSH activities, or to stimulate only one of them while having no effect or an inhibitory effect on the other. They have also found in particular that anti-eCG antibodies able to stimulate the FSH bioactivity of the eCG (while having no effect or a stimulatory effect on the LH bioactivity) greatly enhanced the fertility after artificial insemination.

The present invention relates to an anti-equine chorionic gonadotropin (anti-eCG) polyclonal antibody able to enhance the FSH bioactivity of equine chorionic gonadotropin (eCG), wherein said polyclonal antibody has the following characteristics:
- it recognises native glycosylated eCG and does not recognises deglycosylated eCG;
- it does not recognises equine LH;
- it moderately or strongly cross reacts with the eCG-β subunit;
- it weakly cross reacts with the eCG-α subunit.

The term "polyclonal antibody" includes whole immunoglobulin molecules as well as fragments thereof retaining the specificity of antigen recognition. This comprises in particular Fab, Fab', F(ab)'₂, and Fv fragments.

An antibody is herein defined as "not recognizing" a given form or subunit of eCG, when the percent of cross reactions of said antibody with said form or subunit is equal to or lower than 2% in a competitive ELISA toward native glycosylated eCG. In the same way, an antibody is defined as "weakly cross-reacting" when the percent of cross reactions is higher than 2% and equal to or lower than 10%; as: "moderately cross-reacting" when the percent of cross-reactions is higher than 10% and equal to or lower than 25%, and as: "strongly cross-reacting" when the percent of cross-reactions is higher than 25%.

According to a preferred embodiment of an anti-eCG polyclonal antibody of the invention, it is further able to enhance the LH bioactivity of equine chorionic gonadotropin, and it strongly cross reacts with the eCG-subunit.

Preferably, anti-eCG polyclonal antibodies of the invention do not cross-react with an eCG isoform having a sialic acid content equal to or lower than about 4.7%.

According to preferred embodiments:
- anti-eCG polyclonal antibodies of the invention able to enhance both the LH and the FSH bioactivity of equine chorionic gonadotropin weakly cross-react with an eCG isoform having a sialic acid content of about 9,5%, and strongly cross react with an eCG isoform having a sialic acid content equal to or higher than about 17,3%;
- anti-eCG polyclonal antibodies of the invention able to enhance FSH bioactivity and having no effect on the LH bioactivity of equine chorionic gonadotropin do not cross-react with an eCG isoform having a sialic acid content of about 9,5%, and weakly cross react with an eCG isoform having a sialic acid content equal to or higher than about 17,3%.

Anti-eCG polyclonal antibodies can easily be obtained from plasmas or sera of animals having received at least one injection of eCG. For the production of anti-eCG polyclonal antibodies of the invention, the eCG preparation used for immunization is preferably a preparation having an activity of approximately 500 IU to 4000 IU/mg of protein.

Such a preparation can be obtained from pregnant mare serum by methods known in themselves such as the method disclosed by GOSPODAROWICZ and PAPKOFF, (Endocrinology, 80, 699-702, 1967). Advantageously it can be a commercial preparation of equine chorionic gonadotropin, having an activity of approximately 1500 IU to 4000 IU/mg of protein, such as those used in treatments for inducing and synchronizing oestrus and ovulation. Examples of suitable commercial preparations are CHRONOGEST^{®}-PMSG commercialized by INTERVET, or SYNCHRO-PART^{®}-PMSG commercialized by CEVA.

The dose of eCG used for immunization is generally of about 1,5 to 4,5 µg of protein/kg/injection. For instance, in the case of goats and sheep, a dose of 125 to 350 µg (corresponding to 400 to 600 IU eCG) by animal and can be administered. A single injection is sufficient to induce the production of anti-eCG polyclonal antibodies, that can be recovered as soon as 7 days after the injection; however, since, as indicated above, the maximum anti-eCG polyclonal antibody concentration is reached between 10 and 17 days after injection, it is preferred to recover said antibodies 10 to 25 days after the injection. The interval between the eCG injection and the recovery of the antibodies may however be longer, up to 12 to 15 months: the inventors have indeed noticed that anti-eCG polyclonal antibodies able to stimulate the FSH and/or bioactivity of the eCG were frequently found among the residual anti-eCG antibodies resulting from an eCG injection one year earlier.

Advantageously, anti-eCG polyclonal antibodies can be recovered from sera or plasma of females having received eCG in the course of a standard treatment for inducing and synchronizing oestrus and ovulation. In this case, the females can be nulliparous, primiparous or multiparous at the time of the treatment. One can use females that are treated for the first time, or that have already received the same treatment previously (generally one year before). However they should not have received previous treatments by gonadotropins other than eCG.

The sera or plasma containing the anti-eCG polyclonal antibodies of the invention can be screened on the basis of their properties to stimulate at least the FSH bioactivity of eCG, and preferably the FSH and LH bioactivities of eCG, using for instance the tests disclosed in the examples below. If desired the IgG fraction can be purified, by means known in themselves, for instance by affinity chromatography.

The anti-eCG polyclonal antibodies of the invention can be further purified from the selected plasmas or sera, for instance by affinity chromatography, on the basis of their recognition of native glycosylated eCG and of their lack of recognition of deglycosylated eCG and of equine LH. Advantageously, they can be further purified on the basis of their lack of recognition of eCG isoforms having a sialic acid content equal to or lower than about 4.7%.

The anti-eCG polyclonal antibodies of the invention allow to modulate the biological activity of exogeneously injected eCG. They are useful in particular in the treatments of induction and synchronization of ovulation to diminish the quantity of injected eCG and still obtain a high fertility. Such antibodies are particularly interesting and have applications in particular for animal husbandry, especially for bovines, ovines, caprines, rodents such as murines or rabbits, etc, as well as potentially for human infertility treatment and assisted reproduction techniques where exogenous gonadotropins are intensively used. They are particularly useful in species wherein treatments for inducing and synchronizing oestrus and ovulation are of frequent use, such as ovines and caprines.

The invention also relates to the use of an polyclonal anti-eCG antibody of the invention for preparing a composition for increasing the efficiency of eCG for the induction and synchronization of oestrus and ovulation.

The invention also provides a pharmaceutical composition for use in the induction and synchronization of oestrus and ovulation in a mammal, wherein said composition comprises an antibody of the invention.

According to a preferred embodiment of a pharmaceutical composition of the invention, it further comprises eCG.

The present invention will be further illustrated by the following additional description, which refers to examples illustrating the properties of anti-eCG polyclonal antibodies of the invention. It should be understood however that these examples are given only by way of illustration of the invention and do not constitute in any way a limitation thereof.

### MATERIALS AND METHODS

### Plasma and hormone preparations

Goat plasmas were obtained from a previous study (ROY et al, Biol. Reprod. 60: 805-813, 1999) conducted on females treated in several herds (n=350) and in experimental farm of INRA, Domaine de Galle, Avord, France (n=200). In all cases, females were treated for 11 days with progestogen (vaginal sponge impregnated of 45 mg fluorogestone acetate) and received an intramuscular injection of 500 IU eCG (Syncro-part®, batch 13054A1; CEVA, Libourne, France), 48 h before withdrawal of the sponge. AI was routinely performed 43 h after the sponge was withdrawn. Plasmas samples were collected just before eCG treatment (day 0) and 10 days later (day 10). All plasma samples were stocked at -20 C. Anti-eCG antibodies, present at day 0 and resulting from the previous immune response against eCG one year earlier, were defined as residual anti-eCG antibodies.

For the present study 37 goats were selected which exhibited the highest anti-eCG antibodies concentration at day 0 from 3 µg/ml to 22 µg/ml and at day 10 from 6 µg/ml to 70 µg/ml. The number of previous treatments per goat varies from two to five, depending on the age of the female. The plasma control corresponds to a pool of plasmas from non-eCG treated goats.

Equine chorionic gonadotropin (standard eCG FL 652), eLH, α-eCG and β-eCG subunits and eCG NZY-01 isoforms (LECOMPTE et al., J. Reprod. Fertil. 113:145-150, 1998) were nicely provided by Dr Y. Combarnous and F. Lecompte (INRA, Nouzilly, France). Totally deglycosylated eCG (GRB-VII-128A) was kindly provided by Dr Georges R. Bousfield (Wichita State University, Kansas).

### Anti-eCG antibody affinity purification

Antibodies of selected plasmas were affinity-purified on a HiTrap G protein column (Pharmacia, Uppsala, Sweden) displaying a high binding capacity for immunoglobulins G (IgG) (AKERSTRÖM and BJÖRCK, J. Biol. Chem. 261: 10240-10247, 1986). Briefly, 5 ml of each plasma were submitted to affinity purification, as previously described (ROY et al, Biol. Reprod. 60: 805-813, 1999). The purified antibodies were then stored at -20 C.

### Measurement of anti-eCG antibody concentrations by a quantitative ELISA

The anti-eCG antibody concentrations in plasmas or IgG-purified fractions were measured using a specific quantitative ELISA as previously described (ROY et al, Biol. Reprod. 60: 805-813, 1999). The anti-eCG antibody concentration was expressed in µg/ml of plasma.

### LH in vitro bioassay

The LH *in vitro* bioassay is based on the stimulation of testosterone production by isolated rat Leydig cells as previously described (GUILLOU et al., FEBS Lett. 184: 6-9, 1985). Different concentrations of eCG (100 µl) were preincubated overnight at room temperature, with or no undiluted plasma (9 µl) or the corresponding IgG fraction, added with 91 µl of Leibowitz L15 medium. Leydig cells (15.10⁴ cells in 200 µl L15) were pooled with the preincubated samples for 4 h at 34 C. After centrifugation, the supernatants were stored at -20 C. Testosterone concentrations were assayed by a specific RIA (HOCHEREAU-DE-REVIERS et al., Anim. Reprod. Sci., 23:21-32, 1990).

### FSH in vitro bioassay

The FSH *in vitro* bioassay is based on the stimulation of progesterone production by the Yl cell line derived from a mouse adrenal cortex tumour stably expressing the human FSH receptor (a kind gift from Ares Serono, Geneva, Switzerland) as previously described (KELTON et al. Mol. Cell Endocrinol. 89: 141-151, 1992). Different concentrations of eCG (500 µl), were preincubated overnight at room temperature, either with or no 45 µl of undiluted plasma, or the corresponding IgG fraction, added with 455 µl of culture medium. Then, 15.10⁴ Yl cells were stimulated with 400 µl of preincubated samples for 4 h at 37 C. The media were harvested, boiled, centrifuged and the supernatants were collected and assayed for progesterone by a specific RIA (SAUMANDE et al., Theriogenol. 23: 719-731, 1985).

### Sensitivity of LH and FSH in vitro bioassays

The sensitivities of the two biological assays obtained with Yl cells and Leydig cells were analyzed with eCG ranging from 6 ng/ml to 50 ng/ml. The progesterone secretion varied between 6.93 ng/ml and 33.49 ng/ml and the testosterone secretion between 9.34 ng/ml and 25.67 ng/ml. Progesterone and testosterone secretion start with the same concentration of eCG, i.e. 6 ng/ml. Therefore, the same range of eCG stimulation was used for both *in vitro* biological assays.

### Measurement of anti-eCG plasmas and IgG effects on LH and FSH activities of eCG

In both biological assays, two controls were used. The first one determines the basal level of hormone production by Leydig cells and by Yl cells. The second one determines the level of hormone production by Leydig cells and by Yl cells, in presence of control plasma or of the corresponding IgG fraction devoid of anti-eCG antibodies.

The results are expressed as the percentage of eCG bioactivity : 100 x [(A - B)/(C - D)], A is the amount of progesterone or testosterone secreted during stimulation with defined concentration of eCG preincubated with a plasma or IgG of treated females, B is the amount of progesterone or testosterone secreted during stimulation with preincubated plasma or with IgG of treated females without eCG, C is the amount of progesterone or testosterone secreted during stimulation with a defined concentration of eCG preincubated with a control plasma or with control IgG of untreated females and D is the amount of progesterone or testosterone secreted during stimulation only with control plasma or with control IgG of untreated females.

### Radioligand receptor assay

Only IgG fractions purified from anti-eCG plasmas were analyzed. The ability of anti-eCG IgG to modulate the binding activity of eCG to the rat LH and FSH receptors was analyzed using a radioligand receptor assay (RRA), performed as previously described (GUILLOU et al., 1985, aforementioned). Enriched receptor membrane fractions were prepared from rat testis. For the binding assay, 5 ng/ml to 10 µg/ml of eCG were preincubated overnight at room temperature with 6.5 µg/ml anti-eCG antibodies. Then, 100 µl of each preincubated sample were incubated for 4 h at 34 C, with 50 µl of buffer containing 36 mM CaCl₂, 50 µl of the radioiodinated hormone (40 000 cpm) and 100 µl of testicular membranes. The reaction was stopped by adding 2 ml of cold Tris-HCl 10 mM, pH 7.4 at 4 C and the tubes centrifuged at 3000 g for 40 min. The bound radioactivity was measured in a gamma counter (Packard Instrument, France).

### Affinity measurement of anti-eCG antibodies by Surface Plasmon Resonance (SPR)

Interaction between anti-eCG antibodies and eCG was measured by SPR using a Biacore 1000 system (Biacore International, Uppsala, Sweden). Affinity measurements were performed with diluted plasmas or corresponding affinity-purified IgGs collected at day 0 or day 10. Sensor chips, amine coupling kit and HBS buffer were supplied by Biacore.

eCG (35 µl) prepared at 100 µg/ml in sodium acetate 10 mM pH 4, was covalently coupled either on carboxymethylated dextran CM5 sensor chip for purified IgGs or F1 sensor chip for plasmas analysis, using amine coupling kit as described by manufacturer. F1 sensor chips are well suited for plasma samples because their dextran matrice is shorter than CM5 and reduces non specific binding when diluted plasmas are injected. eCG immobilization level was 6500 RU on CM5 and 1600 RU on F1 chips.

Kinetics measurements of anti-eCG antibodies interaction on coupled eCG were performed with HBS as running (20 µl/min) and diluting buffer, at 23 C. The anti-eCG antibodies concentration of injected IgG fraction (35 µl) ranged from 1.2 nM to 64 nM. Injected plasmas (35 µl) were diluted from 1/10 to 1/80. To prevent non specific interactions on the coated surface, purified IgGs and plasmas were preincubated for 30 min at 37 C in HBS added with 1 mg/ml CM-dextran (Fluka, France) prior injection. The regeneration of coupled surface was performed by injecting NaOH 10 mM (10 µl). The binding curve obtained with control plasma, or purified IgG fraction was used for non specific binding subtraction.

Calculation of kinetic and affinity constants were analyzed using BIA Evaluation software (version 2.2.4). This Biacore software allows calculating kinetic constants (kₒₙ and k_{off}) with standard deviation and a statistical validation test (chi-square). The affinity constant was calculated as the kₒₙ/k_{off} ratio and the dissociation constant as the k_{off}/kₒₙ ratio.

### Specificity of anti-eCG antibodies

The eCG regions recognized by anti-eCG antibodies was determined using a competitive ELISA previously described (MAUREL et al., Biochim. Biophys. Acta 1159: 74-80, 1992). Before the assay, goat plasmas collected at day 0 or day 10 were incubated overnight with increasing concentrations (from 50 ng/ml to 10 µg/ml) of standard eCG FL652, isolated α-eCG and β-eCG subunits, eLH, totally deglycosylated eCG (dg eCG), eCG treated with neuraminidase (eCG SA4 - 1% of sialic acid) and three isoforms of eCG NZY-01 (15) : eCG isoform with 17% of sialic acid (eCG SA1), eCG isoform with 9.5% of sialic acid (eCG SA2), eCG isoform with 4.7% of sialic acid (eCG SA3). The 100% of cross-reaction was obtained with standard eCG FL652.

### Statistical analysis

Average values are represented as means ± SEM. Differences in progesterone and testosterone secretions were determined by Student's *t*-test or ANOVA. Differences were considered significant for *p* values of 0.05 or less.

### EXAMPLE 1: PLASMAS OF eCG TREATED GOATS DIFFERENTLY MODULATE

### THE LH AND FSH BIOACTIVITIES OF eCG.

37 plasmas with high concentrations of anti-eCG antibodies, which are collected just before eCG injection (day 0), and 10 days after injection (day 10), were analyzed for their effects on the LH and FSH bioactivities of eCG, based respectively on the modulation of testosterone and progesterone production, as described in Material and Methods. For this purpose, eCG ranging from 6 ng/ml to 50 ng/ml was preincubated overnight with medium or with plasmas of untreated females (control plasma), or with different plasmas of eCG-treated goats. Then, testosterone and progesterone secretions were measured by RIA after 4 h incubation at 34 C and 37 C, respectively.

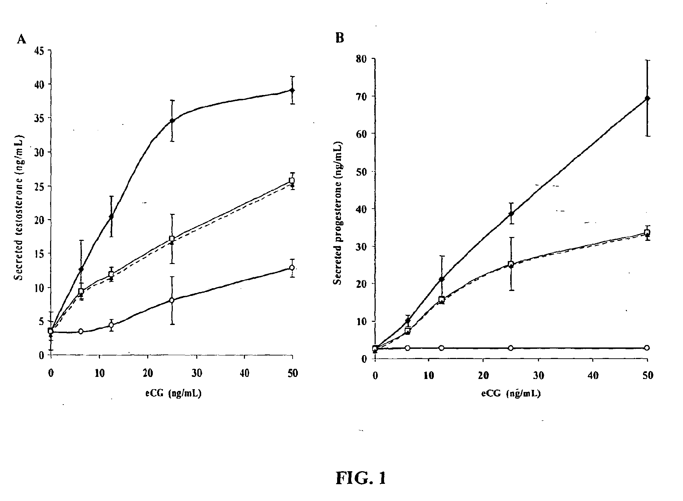

The effects of three representative plasmas of eCG-treated goats on eCG-induced testosterone (A) and progesterone (B) secretions tested in both LH and FSH *in vitro* bioassays are represented in Figure 1.

Legend of Figure 1: A: Testosterone; B: progesterone; eCG preincubated with plasmas of eCG-treated goats (□, ○, ◆), or with a control plasma (A).

Stimulation response curves show three different effects on FSH and LH bioactivities: one of the plasma has an inhibitory effect on both LH and FSH bioactivities (-○-), another one has no effect on both LH and FSH bioactivities (-□-), the third one has a hyperstimulating effect on both LH and FSH bioactivities (-◆-).

For each assay, eCG preincubated with medium or with a control plasma was used as reference. Both controls led to identical testosterone and progesterone secretion when the stimulation was done with a similar concentration of eCG. When eCG was preincubated with plasmas containing anti-eCG antibodies, three different effects on LH and FSH bioactivities of eCG were observed. First, some plasmas displayed no effect on LH and FSH bioactivities and induced the same testosterone and progesterone production as obtained with the control plasma. Secondly, some plasmas displayed an inhibitory effect on LH and/or FSH bioactivities. These plasmas induced lower progesterone and testosterone secretion for all eCG concentrations tested than the control plasma. And in the last case, some plasmas displayed a hyperstimulatory effect on LH and/or FSH bioactivities, leading to higher testosterone and progesterone secretion for all eCG concentrations tested than control plasma. By contrast, when incubated without eCG, all these plasmas had no significant effect on basal secretion of progesterone and testosterone

Among the 37 plasmas containing anti-eCG antibodies that were tested, 10 plasmas had no effect on eCG bioactivities (data not shown) while 27 did modulate eCG bioactivities.

To compare the effects of these 27 latter, LH and FSH bioactivities were determined after overnight preincubation at concentrations of eCG ranging from 12.5 ng/ml to 100 ng/ml. Testosterone and progesterone secretion were measured as above.

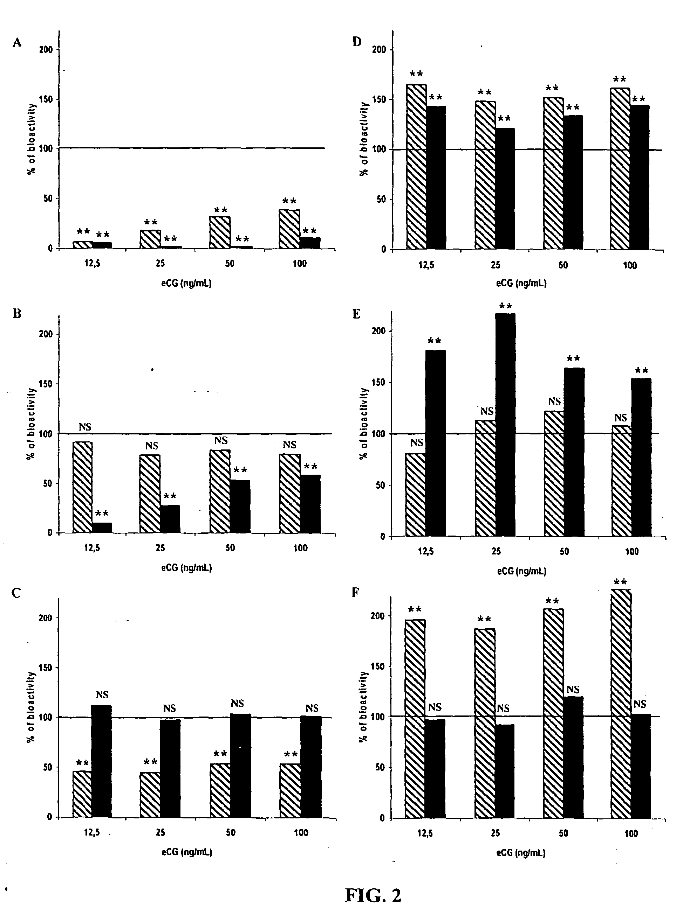

The results are shown in Figure 2:

Results were expressed in percentage of LH (□) or FSH bioactivity (■) of eCG. The dark line corresponding at 100% of bioactivity was obtained with control plasma of untreated female, devoid of anti-eCG antibodies ( - ). The different histograms represent: (A) a plasma displaying an inhibitory effect on both LH and FSH bioactivities; (B) a plasma displaying an inhibitory effect only on the FSH bioactivity; (C) a plasma displaying an inhibitory effect only on the LH bioactivity; (D) a plasma displaying an hyperstimulating effect on both LH and FSH bioactivities; (E) a plasma displaying a hyperstimulating effect only on the FSH bioactivity; and (F) a plasma displaying a hyperstimulating effect only on the LH bioactivity. ** Significant difference in bioactivity percentage (p < 0,05). NS: not significant.

The control plasma used as reference represents 100% of LH and FSH bioactivities. Six sets of plasmas were observed. Three sets included plasmas displaying an inhibitory effect on the eCG bioactivities (n=17): 11 plasmas inhibited up to 10 times both FSH and LH bioactivities (fig 2A), 2 plasmas inhibited up to 10 times only the FSH bioactivity (fig 2B) and 4 plasmas inhibited up to 2 times only the LH bioactivity (fig 2C). Surprisingly, three other sets included plasmas displaying a hyperstimulatory effect on the eCG bioactivities (n=10): 2 plasmas hyperstimulated up to 1.5 times both LH and FSH bioactivities (fig 2D), 4 plasmas hyperstimulated up to 2 times only the FSH bioactivity (fig 2E) and 4 plasmas hyperstimulated up to 2 times only the LH bioactivity (fig 2F). These results demonstrate that plasmas of eCG-treated females containing anti-eCG antibodies can differently modulate only one or both bioactivities of eCG.

### EXEMPLE 2 : IMMUNOGLOBULINS G ARE RESPONSIBLE FOR THE MODULATION OF LH AND FSH BIOACTIVITIES OF eCG.

To ensure that the effects were due to the anti-eCG antibodies and not to plasma contaminants, the effects of different plasmas on the eCG bioactivities were compared with those displayed by their respective purified IgG fraction. One hyperstimulatory plasma and one inhibitory plasma of both bioactivities of eCG were analyzed, as well as one control plasma collected from an untreated female. IgG fractions were obtained by affinity chromatography on Protein G as described in Materials and Methods. Plasmas and their corresponding IgG fractions were tested at the same anti-eCG antibody concentrations. Control and tested IgG fractions were used at a similar concentration of total IgG. Plasmas and the IgG fractions were preincubated overnight with culture medium alone or added with 100 ng/ml eCG. Testosterone and progesterone secretions were measured by RIA.

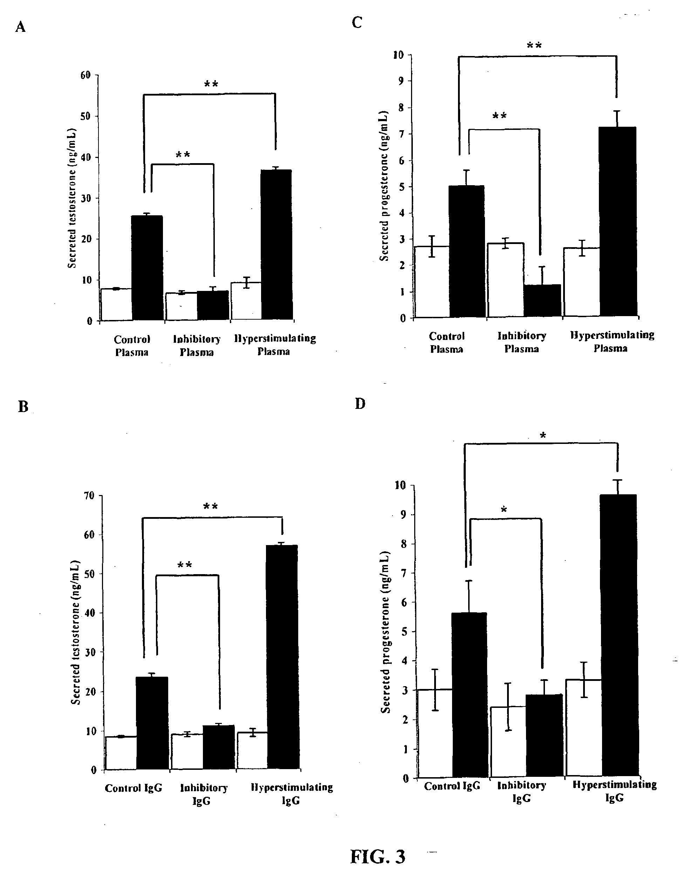

The results are shown in Figure 3.

Legend of Figure 3: A and B: Testosterone; C and D: progesterone; Plasmas and IgG fractions, preincubated with culture medium alone (□) or preincubated with culture medium added with 100 ng/ml eCG (■);** Significant difference in testosterone or progesterone secretion (p < 0,05) or * (p < 0,1). NS: not significant.

When the inhibitory plasma or its purified IgG fraction was preincubated with 100 ng/ml of eCG, testosterone secretion decreased to 28% (fig 3A) and 46% (fig 3B) of the control level respectively, and progesterone secretion to 20% (fig 3C) and 53% (fig 3D) of the control level respectively. When the hyperstimulatory plasma or its purified IgG fraction was preincubated with 100 ng/ml of eCG, testosterone secretion increased to 145% and 242% (fig 3A, B) of the control level respectively, and progesterone secretion to 144% (fig 3C) and 171% (fig 3D) of the control level respectively. The non-IgG counterpart of the plasmas did not modify LH and FSH bioactivities (data not shown).

In conclusion, IgGs preincubated with eCG provoked the same effects on the LH and FSH bioactivities as their corresponding plasmas. These results clearly demonstrate that anti-eCG IgGs present in the plasma are responsible for inhibition or hyperstimulation of LH and FSH bioactivities of eCG.

### EXEMPLE 3 : MODULATION OF LH OR FSH BIOACTIVITY OF eCG BY ANTI-eCG ANTIBODIES DIFFERENTLY AFFECT THE FERTILITY OF TREATED GOATS

For this study, anti-eCG plasmas samples from 37 previously treated goats were used. Plasmas were collected at day 0, just before the eCG injection, and exhibited a high anti-eCG antibodies concentration. The aim was to discriminate which effect of anti-eCG plasmas on LH and FSH bioactivities had the most important impact on the fertility of treated goats after AI. The plasma effect on 12.5 ng/ml of eCG, which roughly corresponds to the quantity of injected eCG (50 µg) taking into account the blood volume of a goat (4 liters), was compared. The effect of anti-eCG plasmas of treated goats on the eCG bioactivities were analyzed according to their fertility result after AI: 11 goats were not pregnant and 26 were pregnant and kidded. Results are summarized in Table 1. Anti-eCG antibody effects on LH or FSH bioactivities of eCG are symbolized by (0) for no effect, (+) for hyperstimulating effect and (-) for inhibitory effect.

**TABLE 1**

| Effects of anti-eCG Abs on eCG bioactivities | FSH (0) | | FSH (+) | | FSH (-) | |
|---|---|---|---|---|---|---|
| Female fertility | Pregnant | Non pregnant | Pregnant | Non pregnant | Pregnant | Non pregnant |
| Number of females | 16 | 5 | 10 | 0 | 0 | 6 |
| (n=37) | (76.2%) | (23.8%) | (100%) | (0%) | (0%) | (100%) |
| | | | | | | |

| Effects of anti-eCG Abs on eCG bioactivities | LH (0) | | LH (+) | | LH (-) | |
|---|---|---|---|---|---|---|
| Female fertility | Pregnant | Non pregnant | Pregnant | Non pregnant | Pregnant | Non pregnant |
| Number of females | 11 | 2 | 10 | 1 | 5 | 8 |
| (n = 37) | (84.6%) | (15.4%) | (90.9%) | (9.1%) | (38.5%) | (61.5%) |

100% of goats having anti-eCG antibodies with hyperstimulatory effect on FSH bioactivity were pregnant. In contrast, none of the goats having anti-eCG antibodies inhibitory of FSH bioactivity was pregnant. However, among the females having anti-eCG antibodies inhibitory of the LH bioactivity, only 38.5% were pregnant. This result contrasts with 84.6% of females pregnant when the anti-eCG antibodies had no effect on the LH bioactivity. It can be noted that these variations are important in comparison with normal pregnancy rate when goats are injected with eCG for the first time which is in average 65%. These results demonstrate that plasmas displaying a hyperstimulatory effect on both bioactivities only came from pregnant goats, and plasmas displaying an inhibitory effect on both bioactivities only came from non pregnant goats. This study explains the lack of pregnancy of treated females due to an inhibition of eCG bioactivities by anti-eCG antibodies, especially antibodies inhibitory of the FSH bioactivity. These results clearly demonstrate that the FSH bioactivity of eCG is necessary to induce ovulation in a accurate timing to get a significant fertility of treated females.

### EXEMPLE 4 : EFFECTS OF THE IgG FRACTIONS PURIFIED FROM PLASMAS OF eCG-TREATED GOATS, ON eCG BINDING TO THE LH AND FSH RECEPTORS.

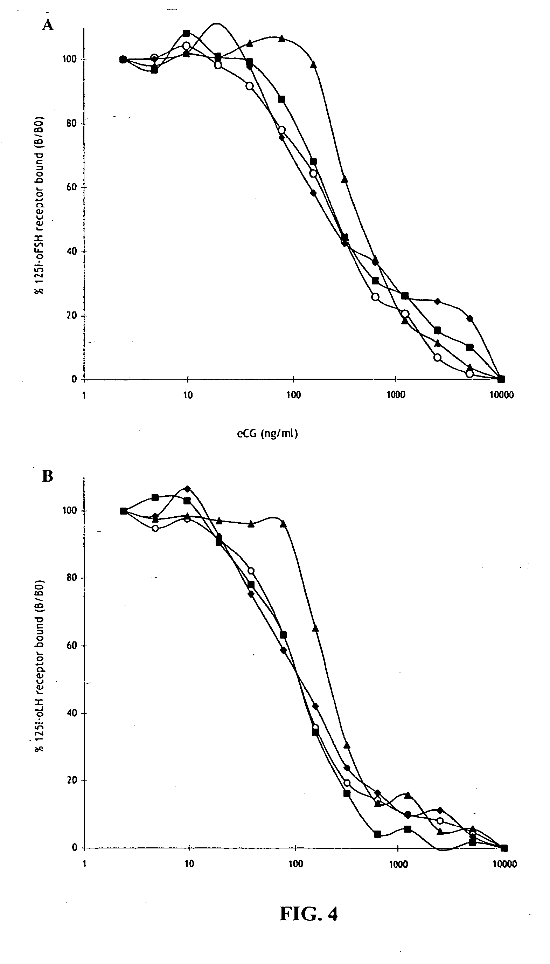

In order to determine if inhibition of the LH and FSH bioactivities was due to inhibition of hormone binding to its receptors, the effects of two IgG fractions inhibitory of both bioactivities, on the binding of eCG to the rat FSH and LH receptors (fig 4A, B), were analyzed. Increased concentrations of eCG, ranging from 5 ng/ml to 10 µg/ml were incubated overnight at room temperature with or without anti-eCG IgG fractions. Three representative purified IgG fractions having different effects on both bioactivities of eCG were tested: two IgG fractions inhibitory of both bioactivities, and an IgG fraction hyperstimulatory of both bioactivities. After 4 h incubation, the radioactivity bound to the FSH-R or to the LH-R was measured.

The results are represented in Figure 4.

Legend of Figure 4: Radioactivity bound to the FSH-R (A) or to the LH-R (B); eCG incubated without anti-eCG IgG fractions (-◆-); eCG incubated with two IgG fractions inhibitory of both bioactivities (-▲-, -■-); eCG incubated with an IgG fraction hyperstimulatory of both bioactivities (-o-).

The first inhibitory IgG fraction elicited 48% and 45% of inhibition of binding to the FSH and LH receptors respectively. The second inhibitory IgG fraction unaltered the binding of eCG to both receptors, since the binding curve obtained was similar with eCG preincubated with medium alone. Similarly, we investigated the effect of an IgG fraction hyperstimulatory of both eCG bioactivities, which did not impair the binding of eCG to the FSH and LH receptors.

### EXEMPLE 5 : AFFINITY FOR eCG OF THE PLASMAS CONTAINING ANTI-eCG ANTIBODIES OR IgG FRACTIONS.

To investigate whether the anti-eCG antibody effects on eCG bioactivities correlated with their affinity for eCG, the affinity for eCG of plasmas of eCG-treated females or of their purified IgG counterpart, was determined by surface plasmon resonance (SPR). Kinetic analysis were conducted on binding curves corrected for non-specific signal by subtracting the reference curve obtained with a control plasma or its corresponding purified IgG fraction on immobilized eCG. Affinity was measured either with purified IgG fractions or with diluted plasmas when sample volumes were limiting. First, we verified that plasmas and their corresponding purified IgG fractions yielded similar kinetic constants (data not shown). The binding curves of plasmas containing anti-eCG antibodies or IgG fractions were compared. The results obtained show strikingly different interaction kinetics, whatever the effect on eCG bioactivities was.

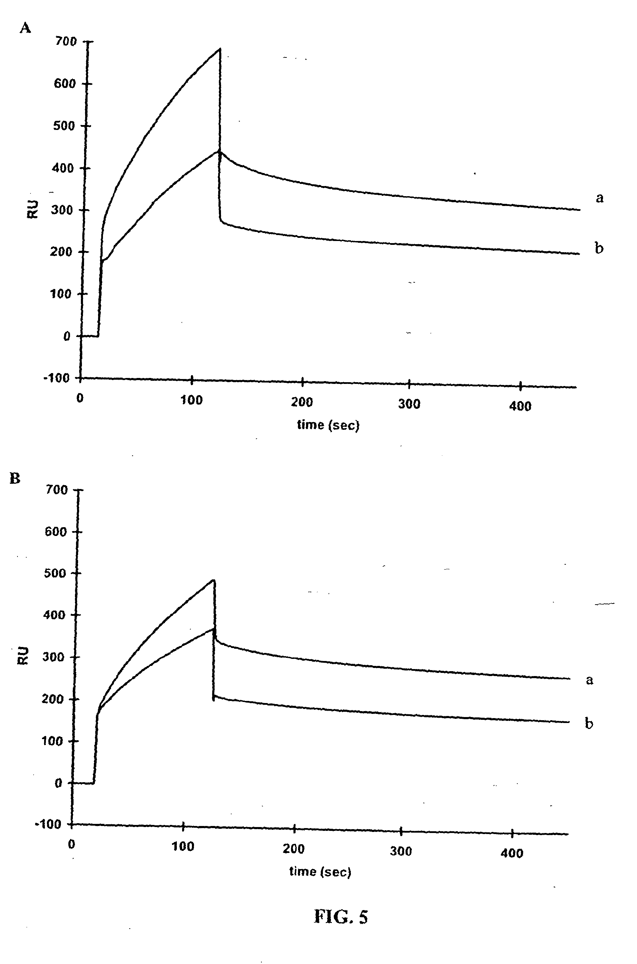

Figure 5 shows the interaction of one plasma containing anti-eCG antibodies without effect on eCG bioactivities (A) and one plasma containing anti-eCG antibodies hyperstimulatory of FSH bioactivity (B). Each plasma was injected at 5.5 nM (a) and 2.7 nM (b) of anti-eCG antibody. Both plasmas strongly interacted, with a maximal level of 688 and 490 RU respectively, and both displayed a slow rate of dissociation (21% after 6 mn) suggesting that the anti-eCG antibodies stably complexed to coated eCG.

Legend of Figure 5: The sensorgrams (A) show the recognition of eCG by goat polyclonal anti-eCG antibodies, without effect on eCG bioactivities and injected at 5.5 nM (a) and 2.7 nM (b) respectively. The sensorgrams (B) show the recognition of eCG by goat polyclonal anti-eCG antibody, hyperstimulating only of FSH bioactivity and injected at 5.5 nM (a) and 2.7 nM (b) respectively.

Affinity measurements of 4 sets of anti-eCG antibodies are summarized in table 2.

Affinity (K_{A}) and dissociation constants (K_{D}) were calculated from association (kₒₙ) and dissociation rates (k_{off}) which were analyzed using BIA evaluation software. Anti-eCG antibodies effects on eCG bioactivities are symbolized by (0) for no effect, (+) for hyperstimulating effect, and (-) for inhibitory effect.

**TABLE 2**

| Anti-eCG Ab effects on eCG bioactivities | kₒₙ (M⁻¹, s⁻¹) | k_{off} s⁻¹ | K_{A} M⁻¹ | K_{d} nM |
|---|---|---|---|---|
| FSH (0) LH (0) n = 3 | 1.66 10⁷ | 1.13 10⁻³ | 1.46 10¹⁰ | 0.068 |
| | 1.33 10⁶ | 1.41 10⁻³ | 9.4 10⁸ | 1.06 |
| | 2.2510⁵ | 1.63 10⁻³ | 1.38 10⁸ | 7.24 |
| FSH (-) LH (-) n = 4 | 1.65 10⁶ | 9.16 10⁻⁵ | 1.8 10¹⁰ | 0.05 |
| | 1.89 10⁵ | 1.54 10⁻⁴ | 1.63 10⁹ | 0.61 |
| | 8.67 10⁵ | 1.74 10⁻³ | 4.98 10⁸ | 2 |
| | 2.32 10⁵ | 7.31 10⁻⁴ | 3.17 10⁸ | 3.15 |
| FSH (+) LH (+) n = 2 | 4.87 10⁵ | 3.37 10⁻⁴ | 1.44 10⁹ | 0.69 |
| | 2.01 10⁵ | 9.96 10⁻⁴ | 2 10⁸ | 4.95 |
| FSH (+) LH (0) n = 5 | 1.43 10⁶ | 3.48 10⁻⁴ | 4.1 10⁹ | 0.24 |
| | 1.12 10⁶ | 1.36 10⁻³ | 8.23 10⁸ | 1.21 |
| | 8.99 10⁵ | 3.73 10⁻³ | 2.41 10⁸ | 4.14 |
| | 6.11 10⁵ | 3.29 10⁻⁴ | 1.85 10⁹ | 5.38 |
| | 9.67 10⁴ | 1.29 10⁻³ | 7.4910⁷ | 13.3 |

The results obtained clearly show a high variability in the affinity (K_{A}) and dissociation constants (K_{D}) values, whatever the effects of anti-eCG antibodies on eCG bioactivities. The dissociation constants varied from 0.068 nM to 13.3 nM. It should be noted that most of the tested plasmas or IgGs displayed a high affinity for eCG, with K_{A} value ranging from 10¹⁰ to 10⁸ M⁻¹. Importantly, these results clearly demonstrate that the lack of effect on eCG bioactivities was not due to a low affinity of anti-eCG antibodies, but rather resulted from the localization of the epitopes they recognize on the hormone surface. Likewise, anti-eCG antibodies displaying strong effect on eCG bioactivities did not display the highest affinity constant.

### EXAMPLE 6: SPECIFICITY OF PLASMAS OF eCG-TREATED GOATS

To determine which part of eCG is recognized by each set of anti-eCG antibodies effect, 21 plasmas of eCG-treated females displaying a clear effect on eCG bioactivities were analyzed. Each plasma was preincubated with native eCG, with eCG-α subunit, with eCG-β subunit, with chemically deglycosylated eCG (dg eCG), or with eLH. Cross-reaction was calculated at 50% of displacement obtained with purified native eCG as competitor, by competitive ELISA.

Table 3 summarizes the specificity of the plasmas in relation with their effects on the eCG bioactivities. Anti-eCG antibodies effects are symbolized by (0) for no effect, (+) for hyperstimulating effect, and (-) for inhibitory effect.

**TABLE 3**

| Plasma Size | Plasma effects on eCG bioactivities | Percentage of cross-reaction with the competitors | | | |
|---|---|---|---|---|---|
| | | eCG dg | α eCG | β eCG | eLH |
| n=1 | LH (+) FSH (+) | 0% | 4% | 44.7% | 0% |
| n=1 | LH (0) FSH (+) | 0% | 4% | 20% | 0% |
| n=5 | LH (-) FSH (0) | <1% | <1% | <1% | 0% |
| n=2 | LH (0) FSH (0) | <1% | <3% | 0% | 0% |
| n=1 | LH (+) FSH (0) | 20% | 0% | 0% | 0% |
| n=2 | LH (-) FSH (-) | <20% | <9% | <1.5% | 0% |
| n=8 | LH (-) FSH (-) | <5% | <5% | <1.5% | 0% |
| n=1 | LH (-) FSH (-) | 0% | 1% | 20% | 0% |

Most plasmas weakly recognized isolated eCG-α and -β subunits, and dg eCG. Nineteen plasmas out of 21 cross-reacted with eCG-α below 5% and the 2 others below 9%. Eighteen plasmas out of 21 cross-reacted with eCG-β below 1.5% and the 3 others between 20% to 44.7%. Eighteen plasmas out of 21 did not recognize dg eCG or very weakly, with a cross-reaction <5%, and the 3 others with a cross-reaction <20%. Therefore, plasmas containing anti-eCG Abs preferentially recognized the eCG αβ dimer and seemed to be influenced by the glycannic chains of the dimeric hormone.

We observed that none plasma having a hyperstimulatory effect on FSH bioactivity of eCG recognized dg eCG but they weakly (4%) recognized eCG-α and clearly cross-reacted with eCG-β subunit (between 44.7% and 20%). Plasmas having an inhibitory effect on both bioactivities of eCG weakly recognized dg eCG and eCG-α subunit. None of the plasmas displaying no effect on both bioactivities of eCG recognized eCG-β. For the other sets of plasmas, we did not observe a correlation between the specificity and the modulation of eCG bioactivities by the anti-eCG antibodies.

To confirm these observations, we analysed the cross-reaction with eLH which has the same peptidic structure as eCG but differs by its glycannic composition. We observed that anti-eCG antibodies did not recognize eLH. These results confirm that various anti-eCG antibodies mainly recognized the glycannic moiety of eCG.

The glycannic part of eCG is mainly composed of bi-antenna glycans ending by sialic acids, in contrast with eLH ending mainly by sulphates. Therefore, plasma specificity towards totally deglycosylated eCG and different desialilated isoforms of eCG was next tested.

For each plasma, the percentage of cross-reactivity was measured towards standard eCG, eCG SA1 (eCG isoform with 17% of sialic acids), eCG SA2 (eCG isoform with 9.5% of sialic acids), eCG SA3 (eCG isoform with 4.7% of sialic acids), eCG treated with neuraminidase (eCG SA4 with 1% of sialic acids) and deglycosylated eCG (dg eCG). The 100% of cross-reaction was obtained with standard eCG. The inhibition of anti-eCG antibody binding to coated eCG was measured by a competitive ELISA, after incubation of plasmas with increasing concentrations of each competitor, ranging from 50 ng/ml to 10 µg/ml.

The results are shown in Table 4: the abbreviation "SA" was employed for sialic acids and "ND" for non determined.

These results show that the sialic acid composition of eCG modulates the plasma specificity for eCG (table 4), which decreases in parallel with the sialic acid content. Moreover, none of the five plasma samples that were tested recognizes desialilated eCG (1% of sialic acids).

These data demonstrate the important role of sialic acids in the immunogenicity of eCG. They reinforce the importance of the specific glycannic sequences of eCG, and especially sialic acids, in the humoral immune response induced by eCG treatment.

**TABLE 4**

| Plasma number | Effect on eCG bioactivities | Percentage of cross-reaction with the competitors | | | | | | | |
|---|---|---|---|---|---|---|---|---|---|
| | | α eCG | β eCG | Dg eCG | eCG SA1 | eCG SA2 | eCG SA3 | eCG SA4 | eLH |
| 94019 | LH(+) FSH(+) | 4% | 44.7% | 0% | 32% | 9% | 0% | ND | 0% |
| 94062 | LH(0) FSH(+) | 3% | 20% | 0% | 0.7% | 0% | 0% | 0% | 0% |
| 93080 | LH(-) FSH(+) | 0.6% | 0.3% | 0.5% | 100% | 83% | 48% | ND | 0% |
| 92078 | LH(-) FSH(+) | 5% | 1.5% | 1.5% | 74% | 65% | 9% | 0% | 0% |
| 93027 | LH(0) FSH(+) | 4% | 20% | 0% | ND | ND | ND | ND | 0% |
| 93019 | LH(0) FSH(0) | 2.3% | 0% | 0% | 74% | 19% | 7% | ND | 0% |
| 93059 | LH(-) FSH(0) | 0% | 0% | 0% | 49% | 29% | 23% | ND | 0% |
| 92004 | LH(+) FSH(0) | 0% | 0% | 20% | 87% | 87% | 31% | ND | 0% |
| 91027 | LH(-) FSH(-) | 3% | 1% | 1.6% | 56% | 43% | 32% | 0% | 0% |
| 601 | LH(-) FSH(-) | 4.5% | 1% | 2% | 52% | 40% | 7% | 0% | 0% |
| 638 | LH(-) FSH(-) | 4% | 1% | 3% | 83% | 72% | 60% | 0% | 0% |
| 93028 | LH(-) FSH(-) | 0% | 1.3% | 17% | 80% | 61% | 10% | ND | 0% |
| 92012 | LH(-) FSH(-) | 1% | 20% | 0% | 80% | 35% | 14% | ND | 0% |

## Claims

1. An anti-equine chorionic gonadotropin (anti-eCG) polyclonal antibody able to enhance the FSH bioactivity of chorionic gonadotropin (eCG), wherein said polyclonal antibody has the following characteristics:
- it recognises native glycosylated eCG and does not recognises deglycosylated eCG ;
- it does not recognises equine LH;
- it moderately or strongly cross reacts with the eCG-β subunit;
- it weakly cross reacts with the eCG-α subunit.

2. An anti-eCG polyclonal antibody of claim 1, **characterised in that** it is further able to enhance the LH bioactivity of chorionic gonadotropin, and it strongly cross reacts with the eCG-β subunit.

3. An anti-eCG polyclonal antibody according to anyone of claims 1 or 2 for increasing the efficiency of eCG for the induction and synchronization of oestrus and ovulation.

4. A pharmaceutical composition for use in the induction and synchronization of oestrus and ovulation in a mammal, **characterized in that** it comprises an anti-eCG polyclonal antibody according to anyone of claims 1 or 2.

5. A pharmaceutical composition according to claim 4, **characterized in that** it further comprises eCG.

## Patentansprüche

1. Polyklonaler Antikörper gegen equines Choriongonadotropin (anti-eCG), der geeignet ist die FSH-Bioaktivität des Choriongonadotropins (eCG) zu verstärken, wobei der polyklonale Antikörper die folgenden Eigenschaften aufweist:
- er erkennt natives glycosyliertes eCG und erkennt deglycosyliertes eCG nicht,
- er erkennt kein equines LH;
- er kreuzreagiert moderat oder stark mit der eCG-β-Untereinheit;
- er kreuzreagiert schwach mit der eCG-α-Untereinheit.

2. Polyklonaler anti-eCG-Antikörper nach Anspruch 1, **dadurch gekennzeichnet, dass** er ferner geeignet ist die LH-Bioaktivität des Choriongonadotropins zu verstärken, und dass er stark mit der eCG-β-Untereinheit kreuzreagiert.

3. Polyklonaler anti-eCG-Antikörper nach einem der Ansprüche 1 oder 2 zum Erhöhen der der Wirksamkeit des eCG für die Einleitung und Synchronisierung des Östrus und der Ovulation.

4. Pharmazeutische Zusammensetzung zur Verwendung bei der Einleitung und Synchronisierung des Östrus und der Ovulation in einem Säugetier, **dadurch gekennzeichnet, dass** er einen polyklonalen anti-eCG-Antikörper nach einem der Ansprüche 1 oder 2 umfasst.

5. Pharmazeutische Zusammensetzung nach Anspruch 4, **dadurch gekennzeichnet, dass** sie ferner eCG umfasst.

## Revendications

1. Anticorps polyclonal anti-gonadotrophine chorionique équine (anti-eCG) capable d'amplifier la bioactivité FSH de la gonadotrophine chorionique (eCG), dans laquelle ledit anticorps polyclonal a les caractéristiques suivantes :
- il reconnaît une eCG glycosylée naturelle et ne reconnaît pas une eCG déglycosylée ;
- il ne reconnaît pas la LH équine ;
- il présente une réaction croisée modérée ou forte avec la sous-unité β de l'eCG ;
- il présente une faible réaction croisée avec la sous-unité α de l'eCG.

2. Anticorps polyclonal anti-eCG, selon la revendication 1, **caractérisé en ce qu'**il est capable en outre d'amplifier la bioactivité LH de la gonadotrophine chorionique et qu'il présente une forte réaction croisée avec la sous-unité β de l'eCG.

3. Anticorps polyclonal anti-eCG, selon l'une quelconque des revendications 1 ou 2, destiné à augmenter l'efficacité de l'eCG pour l'induction et la synchronisation de l'oestrus et de l'ovulation.

4. Composition pharmaceutique destinée à être utilisée dans l'induction et la synchronisation de l'oestrus et de l'ovulation chez un mammifère, **caractérisée en ce qu'**elle comprend un anticorps polyclonal anti-eCG selon l'une quelconque des revendications 1 ou 2.

5. Composition pharmaceutique, selon la revendication 4, **caractérisée en ce qu'**elle comprend en outre de l'eCG.
